(19) Europäisches Patentamt
European Patent Office
Office européen des brevets

(11) Publication number: **0 110 570 B1**

(12) # EUROPEAN PATENT SPECIFICATION

(45) Date of publication of patent specification: **03.02.88**

(21) Application number: **83306569.1**

(22) Date of filing: **28.10.83**

(51) Int. Cl.$^4$: **C 07 D 249/08,** A 01 N 43/653, A 61 K 31/41, C 07 D 401/06

(54) **Triazole antifungal agents.**

(30) Priority: **02.11.82 GB 8231306**

(43) Date of publication of application: **13.06.84 Bulletin 84/24**

(45) Publication of the grant of the patent: **03.02.88 Bulletin 88/05**

(84) Designated Contracting States: **BE DE FR GB IT LU NL**

(58) References cited:
**EP-A-0 043 419**
**EP-A-0 044 605**
**EP-A-0 046 633**
**EP-A-0 047 594**
**EP-A-0 052 424**
**EP-A-0 052 425**
**EP-A-0 060 223**
**EP-A-0 061 789**
**EP-A-0 061 798**

**The file contains technical information submitted after the application was filed and not included in this specification**

(73) Proprietor: **Pfizer Limited**
**Ramsgate Road**
**Sandwich Kent CT13 9NJ (GB)**
(84) **GB**

(73) Proprietor: **Pfizer Corporation**
**Calle 15 1/2 Avenida Santa Isabel**
**Colon (PA)**
(84) **BE DE FR IT LU NL**

(72) Inventor: **Richardson, Kenneth, Dr.**
**48 St. Stephens Hill**
**Canterbury Kent (GB)**
Inventor: **Whittle, Peter John, Dr.**
**5 Winchester Gardens**
**Canterbury Kent (GB)**

(74) Representative: **Wood, David John et al**
**Pfizer Limited Ramsgate Road**
**Sandwich Kent CT13 9NJ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

(56) References cited:
**EP-A-0 061 835**
**EP-A-0 061 840**
**EP-A-0 063 099**
**EP-A-0 070 798**

**Deutsche A. Zeitung N. 41/8/10/81 pages 2209 and 2210**

**Description**

This invention relates to novel triazole derivatives which have antifungal activity and are useful in the treatment of fungal infections in animals, including humans, and as agricultural fungicides.

EP-A-0060223 describes compounds of the formula:

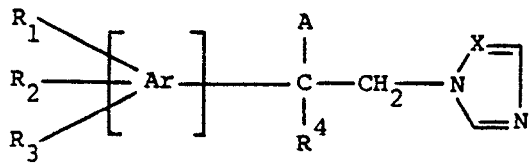

(VI)

where Ar is e.g. phenyl, X is e.g. N, $R_1$, $R_2$ and $R_3$ are each H or a stated substituent, $R^4$ is e.g. alkyl, and A is e.g. C1, Br or I. Although the compounds are synthetic intermediates, they are also described as having fungicidal activity against harmful fungi of the families Fungi imperfecti, Basidiomycetes and Ascomycetes.

According to the invention, there are provided compounds of the formula:—

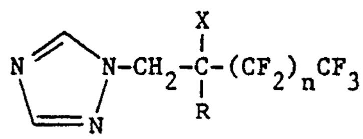

(I)

where R is phenyl optionally substituted by 1 to 3 substituents each independently selected from F, C1, Br, I, $CF_3$, $C_1$—$C_4$ alkyl and $C_1$—$C_4$ alkoxy, or R is a 5-chloropyrid-2-yl group; X is F, C1 or Br; and n is zero or an integer of from 1 to 5; and their pharmaceutically and agriculturally acceptable salts.

$C_3$ and $C_4$ alkyl and alkoxy groups can be straight or branched chain.

The invention also provides a pharmaceutical composition comprising a compound of the formula (I) or a pharmaceutically acceptable salt thereof, together with a pharmaceutically acceptable diluent or carrier. The invention further provides a compound of the formula (I) or a pharmaceutically acceptable salt thereof, for use as a medicament.

The invention yet further provides a fungicidal composition for agricultural use, comprising a compound of the formula (I), or an agriculturally acceptable salt thereof, together with an agriculturally acceptable diluent or carrier.

The invention yet further provides a method of treating a plant or seed having a fungal infection, which comprises contacting said plant or seed, or the locus of said plant, with an effective amount of a compound of the formula (I) or an agriculturally acceptable salt thereof.

When R is said optionally substituted phenyl group, it is preferably phenyl substituted by 1 to 3 substituents, more preferably 1 or 2 substituents, each independently selected from F, C1, Br, I and $CF_3$. The more preferred individual groups represented by R are 4-fluorophenyl, 4-chlorophenyl, 4-bromophenyl, 4-iodophenyl, 4-trifluoromethylphenyl, 2-chlorophenyl, 2-fluorophenyl, 2,4-dichlorophenyl, 2,4-difluorophenyl, 2,5-difluorophenyl, 2-fluoro-4-chlorophenyl, 2,4,6-trifluorophenyl and 4-bromo-2,5-difluorophenyl. Most preferably, R is 2,4-difluorophenyl, 4-chlorophenyl or 4-fluorophenyl. "n" is preferably 0, 1 or 2. "n" is most preferably 0.

X is preferably C1.

The compounds of the formula (I) can be prepared by the halogenation of the corresponding hydroxy compounds of the formula:—

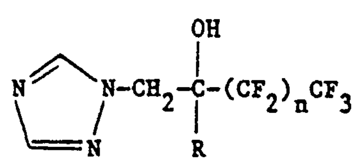

(II).

where R and n are as defined for formula (I).

This halogenation is carried out according to conventional procedures, e.g. using $SOC1_2$, $SOBr_2$ or diethylaminosulphur trifluoride ($Et_2NSF_3$), as appropriate.

In a typical procedure utilising thionyl chloride or bromide, the alkanol (II) in a suitable solvent, e.g. dry acetonitrile, is reacted at up to reflux temperature with thionyl chloride or bromide in the presence of a base, e.g. imidazole. The reaction may proceed to completion at room temperature although in some cases heating at up to reflux temperature may be necessary to accelerate the reaction. The product can be recovered and purified by conventional procedures.

The reaction using diethylaminosulphur trifluoride is typically carried out at about 0°C in methylene chloride as the solvent.

The starting materials of the formula (II) are the subject of our copending U.K. patent applications nos. 8223459 and 8231309 filed on, respectively, 14th August and 2nd November, 1982, and can be prepared by the methods described therein.

These methods for compounds in which m is zero include reacting an oxirane of the formula:—

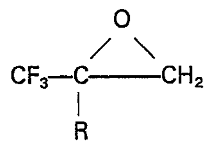

(III)

where R is as defined for formula (I),
with 1,2,4-triazole, preferably in the presence of a base, e.g. $K_2CO_3$. Alternatively an alkali metal salt of 1,2,4-triazole can be used, preparable e.g. from 1,2,4-triazole and NaH. Typically the reaction is carried out by heating the reactants together at up to 120°C in a suitable organic solvent, e.g. dimethylformamide, for up to about 24 hours. The product can then be isolated and purified conventionally.

The oxiranes (III) are obtainable conventionally, generally from the ketones of the formula:—

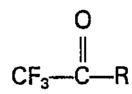

(IV)

This can be achieved by the reaction of (IV) with dimethyloxosulphonium methylide prepared from trimethylsulphoxonium iodide and either (a) sodium hydride in dimethylsulphoxide, or (b) cetrimide (cetyltrimethylammonium bromide) and sodium hydroxide in a mixture of water and toluene or water and 1,1,1-trichloroethane. The reaction using sodium hydride is typically carried out by stirring sodium hydride with trimethylsulphoxonium iodide at, say, room temperature. Dimethylsulphoxide (DMSO) is then added dropwise and the mixture stirred for about 30 minutes, after which time the ketone (IV) is added in DMSO. The desired product is generally obtained by stirring at room temperature for about an hour. The reaction using cetrimide is typically achieved by stirring the ketone (IV), trimethylsulphoxonium iodide and cetrimide in a mixture of 1,1,1-trichloroethane and aqueous sodium hydroxide solution for about 2 hours at, say, 70-100°C. Whilst in either case the oxirane product (III) can be isolated if desired, it is often more convenient to convert this *in situ* to the desired product.

The ketones (IV) are either known compounds or can be prepared conventionally, e.g.:—

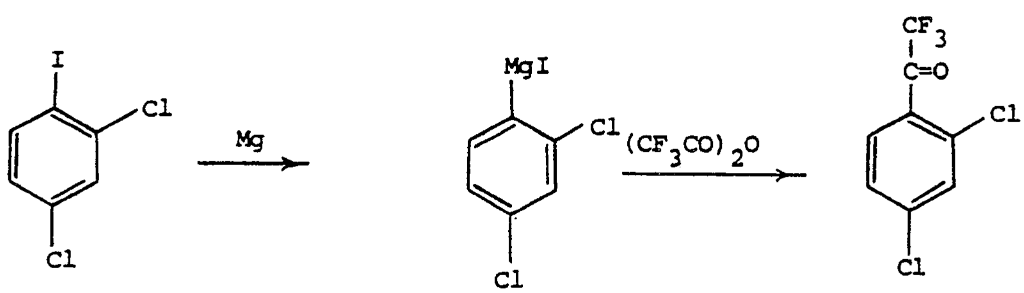

The compounds of the formula (II) in which m is zero can also be prepared as follows:—

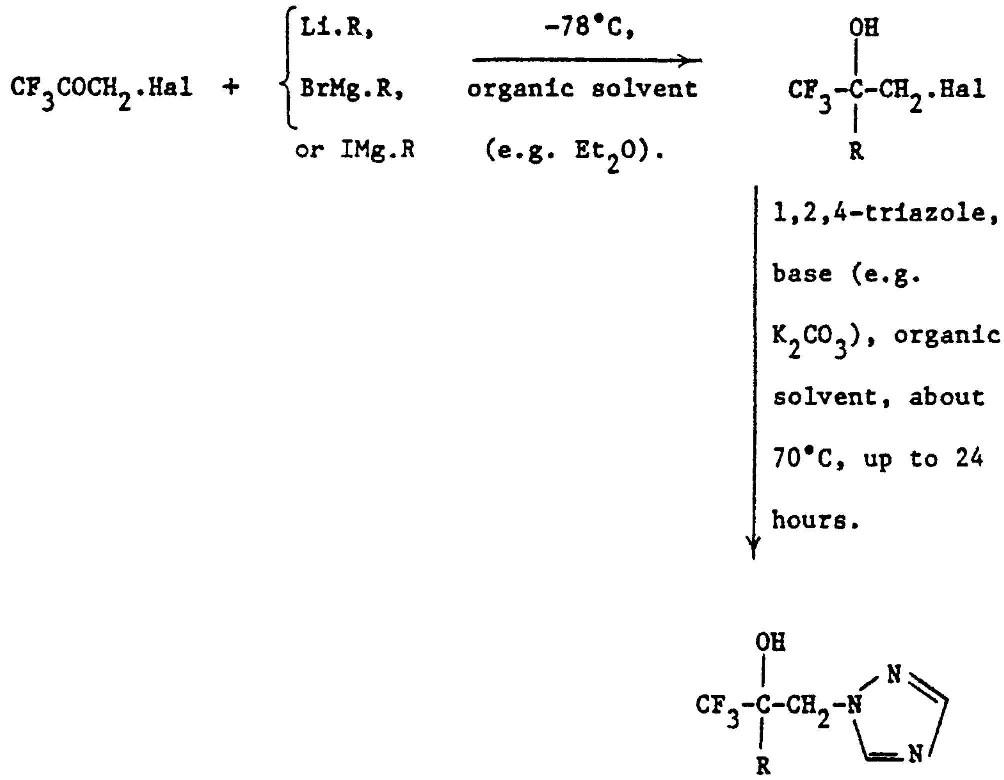


"Hal" = Cl or Br.

Again an alkali metal salt of the triazole can be used in place of 1,2,4-triazole/$K_2CO_3$.

In a typical procedure, the halo-ketone and the lithio or Grignard reagent are stirred together in e.g. diethylether at −78°C for about one hour. The intermediate halo-alkanol can then be recovered conventionally if necessary. The halo-alkanol and 1,2,4-triazole are then heated in e.g. dimethylformamide at, say, 50—130°C, in the presence of a base such as potassium carbonate for up to about 24 hours. The product can then be recovered in a conventional manner.

The compounds of the formula (II) in which n is an integer of from 1 to 5 can be prepared as follows:—

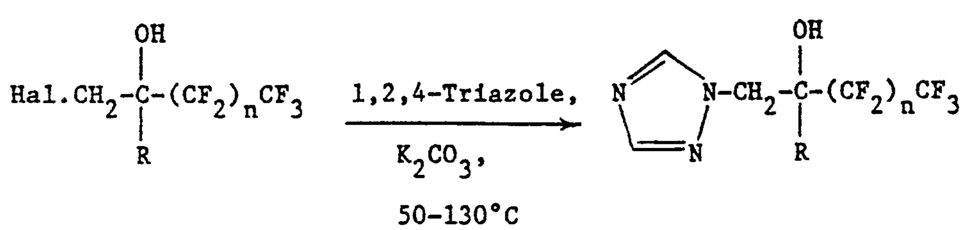


[Hal = Cl or Br and n is 1 to 5].

The reaction is typically carried out by heating in dimethylformamide at up to 130°C for up to, say, 24 hours. Again the product can be recovered in a conventional manner. Again the starting materials are preparable conventionally, e.g.,

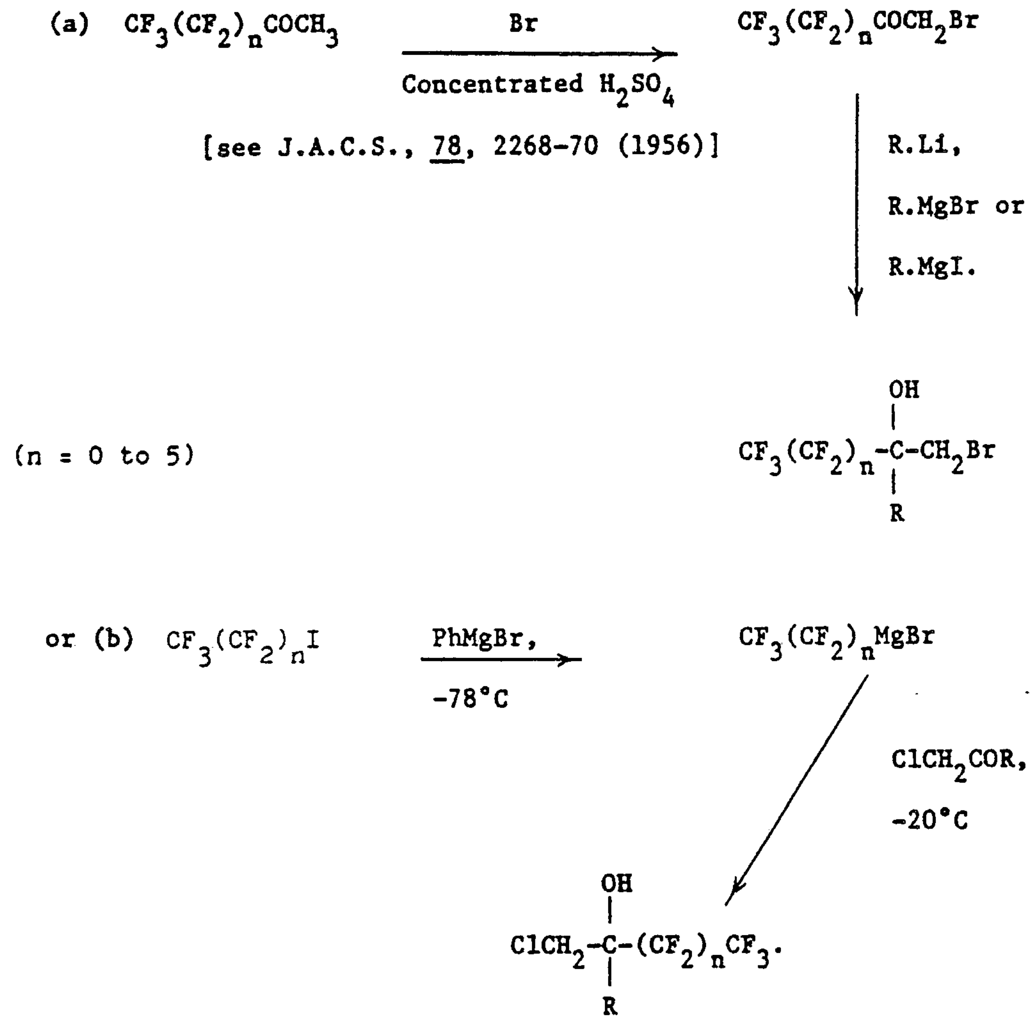


(n = 1 to 5).

All the compounds of the formula (I) contain an optically active centre and the invention includes both the resolved and unresolved forms.

Pharmaceutically acceptable acid addition salts of the compounds of the formula (I) are those formed from strong acids which form non-toxic acid addition salts, such as hydrochloric, hydrobromic, sulphuric, oxalic and methanesulphonic acids.

The salts may be obtained by conventional procedures, e.g. by mixing solutions containing equimolar amounts of the free base and desired acid, and the required salt is collected by filtration, if insoluble, or by evaporation of the solvent.

The compounds of the formula (I) and their pharmaceutically acceptable salts are antifungal agents, useful in combating fungal infections in animals, including humans. For example they are useful in treating topical fungal infections in man caused by, among other organisms, species of *Candida, Trichophyton, Microsporum* or *Epidermophyton,* or in mucosal infections caused by *Candida albicans* (e.g. thrush and vaginal candidiasis). They can also be used in the treatment of systemic fungal infections caused by, for example, *Candida albicans, Cryptococcus neoformans, Aspergillus fumigatus, Coccidioides, Paracoccidioides, Histoplasma* or *Blastomyces.*

The *in vitro* evaluation of the antifungal activity of the compounds can be performed by determining the minimum inhibitory concentration (m.i.c.) which is the concentration of the test compounds in a suitable medium at which growth of the particular micro-organism fails to occur. In practice, a series of agar plates, each having the test compound incorporated at a particular concentration is inoculated with a standard culture of, for example, *Candida albicans* and each plate is then incubated for 48 hours at 37°C. The plates are then examined for the presence or absence of growth of the fungus and the appropriate m.i.c. value is noted. Other micro-organisms used in such tests can include *Cryptococcus neoformans,*

*Aspergillus fumigatus, Trichophyton* spp; *Microsporum* ssp; *Epidermophyton floccosum, Coccidioides immitis* and *Torulopsis glabrata.*

The *in vivo* evaluation of the compounds can be carried out at a series of dose levels by intraperitoneal or intravenous injection or by oral administration, to mice which are inoculated with a strain of *Candida albicans.* Activity is based on the survival of a treated group of mice after the death of an untreated group of mice following 48 hours observation. The dose level at which the compound provides 50% protection against the lethal effect of the infection ($PD_{50}$) is noted.

For human use, the antifungal compounds of the formula (I) can be administered alone, but will generally be administered in admixture with a pharmaceutical carrier selected with regard to the intended route of administration and standard pharmaceutical practice. For example, they can be administered orally in the form of tablets containing such excipients as starch or lactose, or in capsules or ovules either alone or in admixture with excipients, or in the form of elixirs or suspensions containing flavouring or colouring agents. They can be administered parenterally, for example, intravenously, intramuscularly or subcutaneously. For administration by injection, they are best used in the form of a sterile aqueous solution which may contain other substances, for example, enough salts or glucose to make the solution isotonic with blood.

For oral and parenteral administration to human patients, the daily dosage level of the antifungal compounds of the formula (I) will be from 0.1 to 5 mg/kg (in divided doses) when administered by either the oral or parenteral route. Thus tablets or capsules of the compounds will typically contain from 5 mg to 0.5 g of active compound for administration singly or two or more at a time as appropriate. The physician in any event will determine the actual dosage which will be most suitable for an individual patient and it will vary with the age, weight and response of the particular patient. The above dosages are exemplary of the average case; there can, of course, be individual instances where higher or lower dosage ranges are merited, and such are within the scope of this invention.

Alternatively, the antifungal compounds of formula (I) can be administered in the form of a suppository or pessary, or they may be applied topically in the form of a lotion, solution, cream, ointment or dusting powder. For example, they can be incorporated into a cream consisting of an aqueous emulsion of polyethylene glycols or liquid paraffin; or they can be incorporated, at a concentration between 1 and 10%, into an ointment consisting of a white wax or white soft paraffin base together with such stabilizers and preservatives as may be required.

The compounds of the formula (I) and their salts also have activity against a variety of plant pathogenic fungi, including for example various rusts, mildews and moulds, and the compounds are thus useful for treating plants and seeds to eradicate or prevent such diseases.

The *in vitro* evaluation of the activity of the compounds against plant fungi can be determined by measuring their minimum inhibitory concentrations in the same way as previously described except that the places are incubated at 30°C for 48 hours or longer before being examined for the presence or absence of growth.

Micro-organisms used in such tests include *Cochliobolus carbonum, Pyricularia oryzae, Glomerella cingulata, Penicillium digitatum, Botrytis cinerea* and *Rhizoctonia solani.*

For agricultural and horticultural purposes the compounds and their agriculturally acceptable salts are preferably used in the form of a composition formulated as appropriate to the particular use and purpose desired. Thus the compounds may be applied in the form of dusting powders, or granules, seed dressings, aqueous solutions, dispersions or emulsions, dips, sprays, aerosols or smokes. Compositions may also be supplied in the form of dispersible powders, granules or grains, or concentrates for dilution prior to use. Such compositions may contain such conventional carriers, diluents or adjuvants as are known and acceptable in agriculture and horticulture and they are manufactured in accordance with conventional procedures. The compositions may also incorporate other active ingredients, for example, compounds having herbicidal or insecticidal activity or a further fungicide. The compounds and compositions can be applied in a number of ways, for example they can be applied directly to the plant foliage, stems, branches, seeds or roots or to the soil.

The following Examples illustrate the invention. All temperatures are in °C:—

Example 1

Preparation of 1-[2-chloro-2-(2,4-difluorophenyl)-3,3,3-trifluoropropyl]-1H-1,2,4-triazole

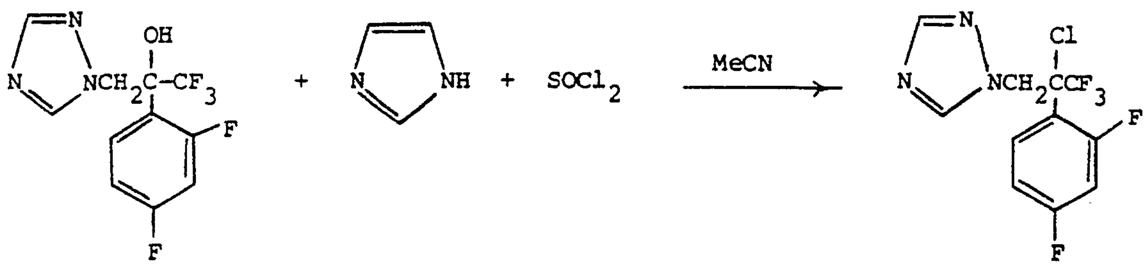

Imidazole (0.53 g, 7.8 m.mole) was dissolved in dry acetonitrile (4 ml) and thionyl chloride (0.3 ml, 4.1 m.mole) was added dropwise over 2 minutes followed by a solution of 2-(2,4-difluorophenyl)-1-(1H-1,2,4-triazol-l-yl)-3,3,3-trifluoropropan-2-ol (0.389 g, 1.25 m.mole) in dry acetonitrile (12 ml). The mixture was stirred at room temperature for 24 hours and then heated to 70°C for ½ hour. The cooled mixture was then poured into sodium bicarbonate solution and extracted with dichloromethane (3 × 100 ml). The combined dichloromethane extracts were washed once with water, dried over magnesium sulphate and evaporated. Chromatography of the residue on silica (230—400 mesh), eluting with 40:60 (by volume) ethyl acetate:hexane gave, after recrystallisation from ethyl acetate/hexane, the title compound, 170 mg (41%,) m.p. 87-88°.

Analysis %:—

| | |
|---|---|
| Found: | C,42.4; H,2.3; N,13.4. |
| $C_{11}H_7ClF_5N_3$ requires: | C,42.4; H,2.3; N,13.5 |

Examples 2 to 5

The compounds of these Examples were prepared similarly to Example 1 from appropriate starting materials. In Examples 2 and 3, thionyl chloride was added to a solution of the starting alcohol and imidazole, and in Example 2, water and ethyl acetate were used in the extraction procedure in place of, respectively, sodium bicarbonate and dichloromethane.

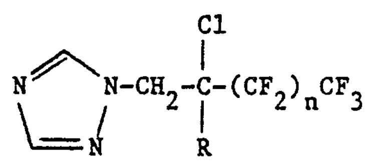

| Example No. | R | n | % Yield | m.p. (°C) | Analysis % (Theoretical in brackets) C | H | N |
|---|---|---|---|---|---|---|---|
| 2 | 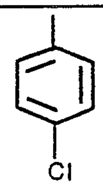 | 0 | 85 | 62—64 | 42.7<br>(42.6 | 2.7<br>2.6 | 13.7<br>13.5) |
| 3 | 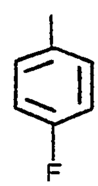 | 0 | 42 | 75—77 | 44.9<br>(45.0 | 2.6<br>2.7 | 14.1<br>14.3) |
| 4 | 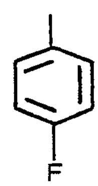 | 1 | 57 | 110—113 | 42.3<br>(41.9 | 2.3<br>2.4 | 12.3<br>12.2) |
| 5 | 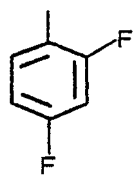 | 2 | 51 | 74—76 | 38.0<br>(37.9 | 1.7<br>1.7 | 10.0<br>10.2) |

Example 6

The following illustrate pharmaceutical compositions for the treatment of fungal infections:—

(a) Capsule: 71 parts by weight of the compound of Example 1 are granulated with 3 parts maize starch and 22 parts lactose and then a further 3 parts maize starch and 1 part magnesium stearate are added. The mixture is regranulated and filled into hard gelatin capsules.

(b) Cream: 2 parts by weight of the compound of Example 1 are dissolved in 10 parts of propylene glycol and mixed into 88 parts of a vanishing cream base.

(c) Pessary: 2 parts by weight of the compound of Example 3 are suspended in 98 parts of a warm liquified suppository base which is poured into moulds and allowed to solidify.

The following Preparations illustrate the preparation of certain starting materials. All temperatures are in °C:—

Preparation 1

Preparation of 2-(2,4-difluorophenyl)-1-(1H-1,2,4-triazol-1-yl)-3,3,3-trifluoropropan-2-ol

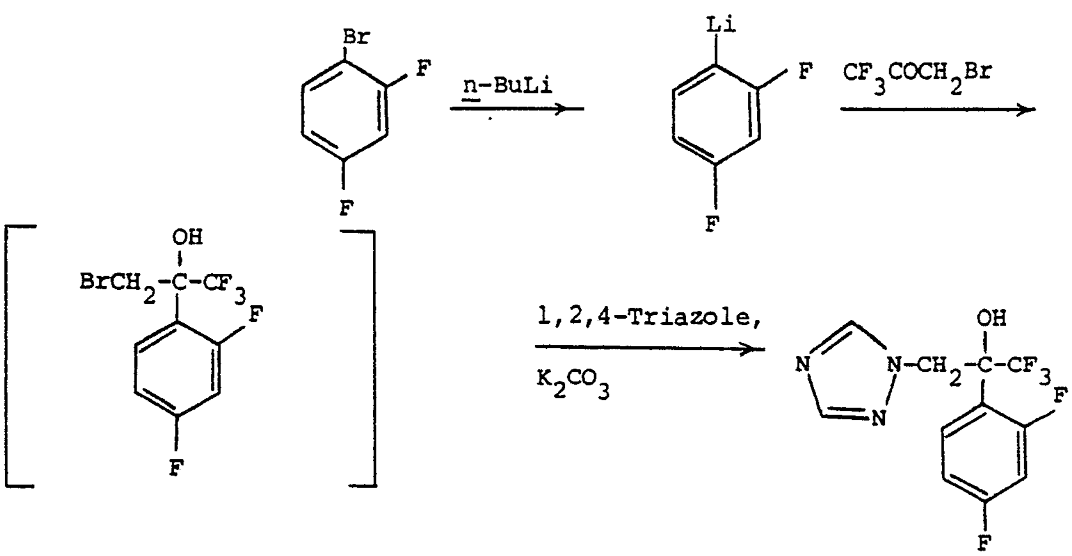


A hexane solution of n-butyllithium (1.55 M, 9.6 ml, 14.9 m.mole) was added to diethylether (6 ml) and the solution was cooled to −78°. A solution of 2,4-difluorobromobenzene (3.03 g, 15.7 m.mole) in diethylether (100 ml) was added dropwise over 15 minutes and the mixture was stirred at −78° for a further 15 minutes. A solution of 1-bromo-3,3,3-trifluoropropan-2-one (2.4 g, 12.6 m.mole) in diethylether (100 ml) was then added dropwise over 15 minutes and the mixture was stirred at −78° for a further 30 minutes. A solution of glacial acetic acid (2 ml) in diethylether (5 ml) was then added, followed by water (15 ml) and the mixture was allowed to warm to 0°. The aqueous layer was separated and washed with diethylether (2 × 30 ml). The combined diethylether extracts were dried over magnesium sulphate, evaporated, and the residual oil was dissolved in dimethylformamide (40 ml). 1,2,4-Triazole (2.5 g, 36.2 m.mole) and anhydrous potassium carbonate (10 g, 72.5 m.mole) were then added to this solution and the mixture was stirred and heated at 70° for 18 hours. The mixture was then cooled, poured into water (150 ml) and extracted with ethyl acetate (3 × 100 ml). The combined ethyl acetate extracts were washed with water (100 ml), dried over magnesium sulphate, and evaporated. The residue was chromatographed on silica (230—400 mesh), eluting with ethyl acetate: hexane, 60:40 by volume, to give, after one recrystallisation from ethyl acetate/hexane, the title compound, 1.7 g (47%), m.p. 110—111°.

| | |
|---|---|
| Analysis %:— | |
| Found: | C,45.2; H,2.7; N,14.6 |
| $C_{11}H_8F_5N_3O$ requires: | C,45.0; H,2.7; N,14.3. |

Preparations 2 and 3

The following compounds were prepared similarly to Preparation 1 using n-butyllithium 4-fluorobromobenzene and either 1-bromo-3,3,3-trifluoropropan-2-one or 1-bromo-3,3,4,4,4-pentafluorobutan-2-one.

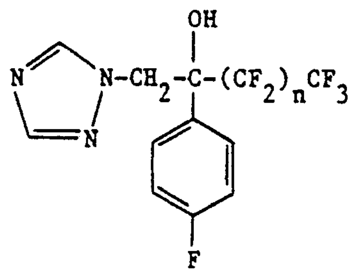

| Preparation No. | n | m.p. (°C) | Analysis % (Theoretical in brackets) C | H | N |
|---|---|---|---|---|---|
| 2 | 0 | 134—136° | 47.8 | 3.2 | 15.5 |
| | | | (48.0 | 3.3 | 15.3) |
| 3 | 1 | 95—97° | 44.6 | 2.8 | 13.3 |
| | | | (44.3 | 2.8 | 12.9) |

Preparation 4

Preparation of 2-(4-chlorophenyl)-1-(1H-1,2,4-triazol-l-yl)-3,3,3-trifluoropropan-2-ol

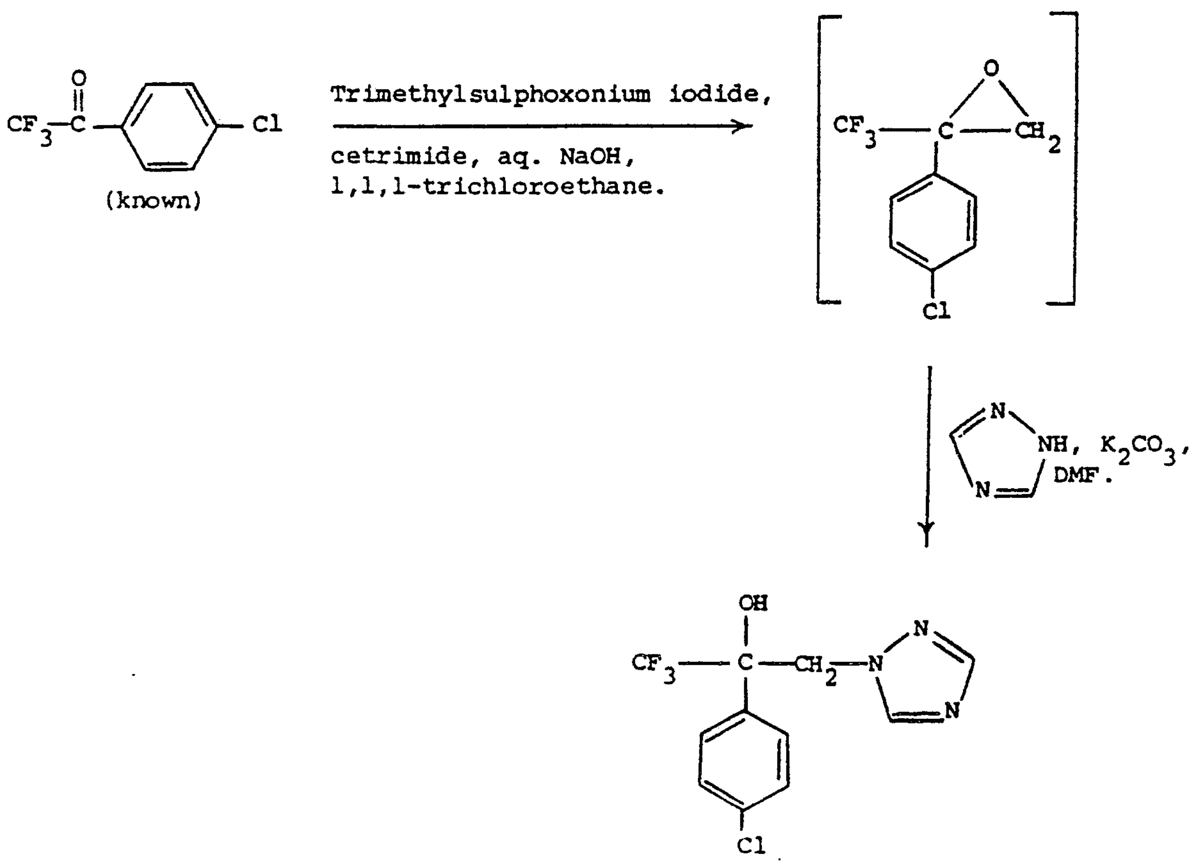


2,2,2-Trifluoro-4'-chloroacetophenone (0.8 g, 3.84 m.mole), trimethylsulphoxonium iodide (1.02 g, 4.6 m.mole) and cetyltrimethylammonium bromide (0.1 g, 0.27 m.mole) were stirred in a mixture of 1,1,1-trichloroethane (40 ml) and 18% sodium hydroxide solution (20 ml) at 75° for 2 hours. The mixture was then allowed to cool, the organic layer was separated, evaporated, and the residue was stirred in dimethylformamide (50 ml) with 1,2,4-triazole (1 g, 14.5 m.mole) and anhydrous potassium carbonate (2 g, 14.5 m.mole) at 90° for 4 hours. The mixture was then allowed to cool, ethyl acetate (100 ml) and water (50 ml) were added and the aqueous layer was separated. The organic layer was washed a further 6 times with water (200 ml in total), dried over magnesium sulphate, and evaporated to give a gum (104 mg) which was chromatographed on silica (230—400 mesh), eluting with ethyl acetate to give as a colourless solid the title compound, 84 mg, (8%). One recrystallisation from dichloromethane/hexane gave colourless crystals, 64 mg, m.p. 117—118°.

| | |
|---|---|
| Analysis %:— | |
| Found: | C,45.4; H,3.1; N,14.8. |
| Required for $C_{11}H_9ClF_3N_3O$: | C,45.2; H,3.1; N,14.4. |

Preparation 5

Preparation of 2-(2,4-difluorophenyl)-3,3,4,4,5,5,5-heptafluoro-1-(1H-1,2,4-triazol-1-yl)-pentan-2-ol

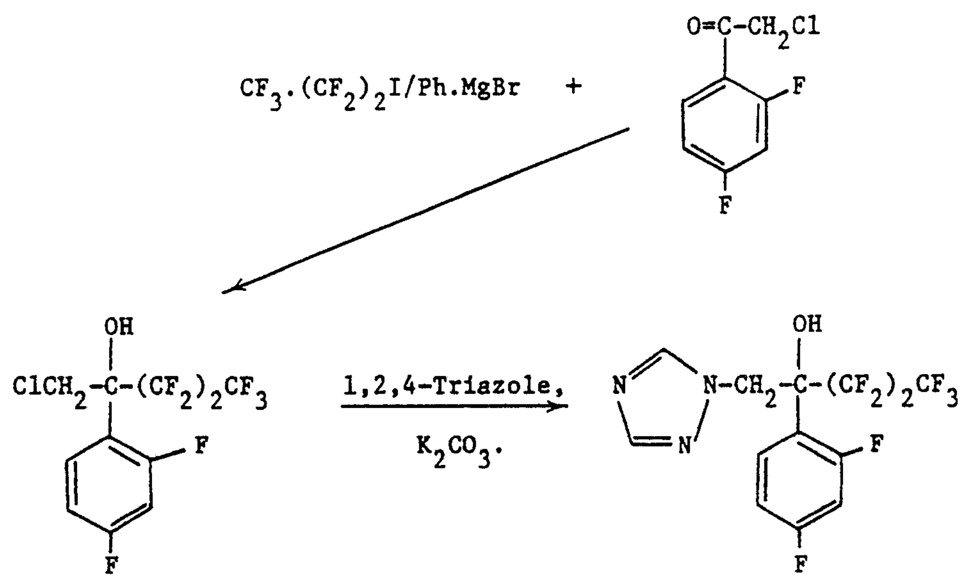


To a solution of heptafluoropropyl iodide (5 g, 0.017 M) in ether (20 ml) cooled to −78° was added phenylmagnesium bromide (5.6 ml. of a 3 molar solution in ether, 0.017 M) dropwise at such a rate that the temperature of the reaction mixture did not exceed −50°. When all the phenylmagnesium bromide had been added, the mixture was stirred at −50° for ½ hour and then cooled again to −78°. A solution of 2-chloro-2′,4′-difluoroacetophenone (3.6 g, 0.019 M) in ether (20 ml) was added dropwise at such a rate that the temperature of the reaction mixture did not exceed −50° and, when the addition was complete, the reaction mixture was allowed to warm to −20° and was stirred at this temperature for 2 hours. A solution of glacial acetic acid (3 ml) in ether (5 ml) was then added, followed by water (15 ml) and the mixture was allowed to warm to about 5°. The aqueous phase was separated and extracted with ether (2 × 50 ml) and the combined ether extracts were dried ($MgSO_4$) and evaporated to give a pale yellow oil (6.7 g). This oil was added to a mixture of 1,2,4-triazole (5.87 g, 0.085 M) and anhydrous potassium carbonate (17.5 g, 0.127 M) in dimethylformamide (60 ml) and this mixture was stirred at a temperature of 80° for 3 hours. The reaction mixture was then cooled, the dimethylformamide was evaporated and the residue was partitioned between water (200 ml) and ethyl acetate (150 ml). The aqueous layer was separated and extracted with ethyl acetate (3 × 150 ml). The combined ethyl acetate extracts were washed successively with aqueous sodium bisulphite and water, dried ($MgSO_4$), evaporated and the residue was flash chromatographed on silica (230—400 mesh), eluting with a mixture of hexane:

isopropylalcohol:0.88 ammonium hydroxide, 80:20:1.5 by volume, to yeild, after one recrystallisation from hexane:dichloromethane, the title compound, 1.51 g (23%), m.p. 128—129°.

| | |
|---|---|
| Analysis %:— | |
| Found: | C,39.8; H,2.0; N,10.6. |
| Required for $C_{13}H_8F_9N_3O$: | C,39.7; H,2.1; N,10.7. |

Using the test method described previously in the text, the following $PD_{50}$ values (mg./kg.) were obtained in mice infected with *Candida albicans*:—

| Compound | | $PD_{50}$ (mg./kg.) |
|---|---|---|
| Product of Example | 1 | 0.15 |
| | 2 | 0.6 |
| | 3 | 0.6 |
| | 4 | < 1.0 |
| | 5 | < 1.0. |

**Claims**

1. A compound of the formula:—

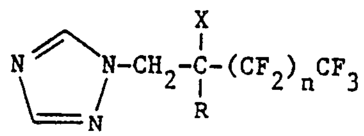

(I)

where R is a phenyl group optionally substituted by 1 to 3 substituents each independently selected from F, C1, Br, I, $CF_3$, $C_1$—$C_4$ alkyl and $C_1$—$C_4$ alkoxy, or R is a 5-chloropyrid-2-yl group; X is F, Cl or Br; and n is zero or an integer of from 1 to 5; or a pharmaceutically or agriculturally acceptable salt thereof.

2. A compound as claimed in claim 1, wherein R is 4-fluorophenyl, 4-chlorophenyl, 4-bromophenyl, 4-iodophenyl, 4-trifluoromethylphenyl, 2-chlorophenyl, 2-fluorophenyl, 2,4 dichlorophenyl, 2,4-difluorophenyl, 2,5-difluorophenyl, 2-fluoro-4-chlorophenyl, 2,4,6-trifluorophenyl or 4-bromo-2,5-difluorophenyl.

3. A compound as claimed in claim 2, wherein R is 2,4-difluorophenyl, 4-chlorophenyl or 4-fluorophenyl.

4. A compound as claimed in any one of the preceding claims, wherein X is Cl.

5. A compound as claimed in any one of the preceding claims, wherein n is 0, 1 or 2.

6. A compound as claimed in claim 5, wherein n is O.

7. A compound of the formula (I) as claimed in any one of claims 1 to 6, or a pharmaceutically acceptable salt thereof, for use as a medicament.

8. A pharmaceutical composition comprising a compound of the formula (I) as claimed in any one of claims 1 to 6, or a pharmaceutically acceptable salt thereof, and a pharmaceutically acceptable diluent or carrier.

9. A fungicidal composition for agricultural use, comprising a compound of the formula (I) as claimed in any one of claims 1 to 6, or an agriculturally acceptable salt thereof, and an agriculturally acceptable diluent or carrier.

10. A method of treating a plant or seed having a fungal infection, which comprises contacting said plant or seed, or the locus of said plant, with an antifungally effective amount of a compound as claimed in any one of claims 1 to 6 or agriculturally acceptable salt thereof, or with a composition as claimed in claim 9.

**Patentansprüche**

1. Verbindung der Formel

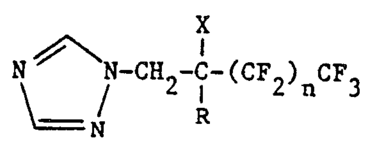

(I)

worin R eine Phenylgruppe darstellt, gegebenenfalls substituiert durch 1 bis 3 Substituenten jeweils unabhängig voneinander ausgewählt unter F, Cl, Br, I, $CF_3$, $C_1$—$C_4$-Alkyl und $C_1$—$C_4$-Alkoxy oder R eine 5-Chloropyrid- 2-yl-Gruppe darstellt; X die Bedeutung von F, Cl oder Br besitzt und n 0 oder eine ganze Zahl von 1 bis 5 darstellt; oder ein pharmazeutisches oder für die Landwirtschaft annehmbares Salz davon.

2. Verbindung gemäß Anspruch 1, worin R 4-Fluorophenyl, 4-Chlorophenyl, 4-Bromophenyl, 4-Iodophenyl, 4-Trifluoromethylphenyl, 2-Chlorophenyl, 2-Fluorophenyl, 2,4-Dichlorophenyl, 2,4-Difluorophenyl, 2,5-Difluorophenyl, 2-Fluoro-4-chlorophenyl, 2,4,6-Trifluorophenyl oder 4-Bromo-2,5-difluorophenyl darstellt.

3. Verbindung gemäß Anspruch 2, worin R 2,4-Difluorophenyl, 4-Chlorophenyl oder 4-Fluorophenyl darstellt.

4. Verbindung gemäß einem der vorgehenden Ansprüche, worin X die Bedeutung von Cl hat.

5. Verbindung gemäß einem der vorgehenden Ansprüche, worin n 0, 1 oder 2 ist.

6. Verbindung gemäß Anspruch 5, worin n 0 ist.

7. Verbindung gemäß Formel (I) gemäß einem der vorgehenden Ansprüche 1 bis 6, oder ein pharmazeutisch annehmbares Salz davon, zur Verwendung als Medikament.

8. Pharmazeutische Zusammensetzung enthaltend eine Verbindung der Formel (I) gemäß einem der Ansprüche 1 bis 6, oder ein pharmazeutisch annehmbares Salz davon, und ein pharmazeutisch annehmbares Verdünnungsmittel oder Träger.

9. Fungizide Zusammensetzung für landwirtschaftliche Zwecke enthaltend eine Verbindung der Formel (I) gemäß einem der Ansprüche 1 bis 6, oder ein in der Landwirtschaft annehmbares Salz davon, und ein in der Landwirtschaft annehmbares Verdünnungsmittel oder Träger.

10. Verfahren zur Behandlung von Pflanzen oder Saatgut mit Pilzbefall, wobei die Pflanze oder das Saatgut oder die Stelle der Pflanze mit einer fungizid wirksamen Menge einer Verbindung gemäß einem der Ansprüche 1 bis 6 oder eines in der Landwirtschaft annehmbaren Salzes davon oder mit einer Zusammensetzung gemäß Anspruch 9 in Berührung gebracht wird.

**Revendications**

1. Composé de formule

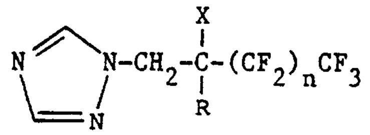

(I)

dans laquelle R est un groupe phényle portant facultativement 1 à 3 substituants, chacun choisi indépendamment entre F, Cl, Br, I, $CF_3$, un groupe alkyle en $C_1$ à $C_4$ et alkoxy en $C_1$ à $C_4$, ou bien R est un groupe 5-chloropyrid-2-yle; X représente F, Cl ou Br; et n est égal à zéro ou est nombre entier de 1 à 5; ou un de ses sels acceptables en pharmacie ou en agriculture.

2. Composé suivant la revendication 1, dans lequel R est un groupe 4-fluorophényle, 4-chlorophényle, 4-bromophényle, 4-iodophényle, 4-trifluorométhylphényle, 2-chlorophényle, 2-fluorophényle, 2,4-dichlorophényle, 2,4-difluorophényle, 2,5-difluorophényle, 2-fluoro-4-chlorophényle, 2,4,6-trifluorophényle ou 4-bromo-2,5-difluorophényle.

3. Composé suivant la revendication 2, dans lequel R est un groupe 2,4-difluorophényle, 4-chlorophényle ou 4-fluorophényle.

4. Composé suivant l'une quelconque des revendications précédentes, dans lequel X représente Cl.

5. Composé suivant l'une quelconque des revendications précédentes, dans lequel n a la valeur 0, 1 ou 2.

6. Composé suivant la revendication 5, dans lequel n a la valeur 0.

7. Composé de formule (I) suivant l'une quelconque des revendications 1 à 6, ou un de ses sels pharmaceutiquement acceptables, destiné à être utilisé comme médicament.

8. Composition pharmaceutique comprenant un composé de formule (I) suivant l'une quelconque des revendications 1 à 6, ou un de ses sels pharmaceutiquement acceptables, et un diluant ou support pharmaceutiquement acceptable.

9. Composition fongicide destinée à être utilisée en agriculture, comprenant un composé de formule (I) suivant l'une quelconque des revendications 1 à 6, ou un de ses sels acceptables en agriculture, et un diluant ou support acceptable en agriculture.

10. Procédé de traitement d'une plante ou de graines présentant une infection fongique, qui consiste à mettre en contact ladite plante ou lesdites graines, ou le milieu de ladite plante, avec une quantité efficace du point de vue antifongique d'un composé suivant l'une quelconque des revendications 1 à 6 ou d'un de ses sels acceptables en agriculture, ou avec une composition suivant la revendication 9.